# EUROPEAN PATENT APPLICATION

(11) **EP 4 699 589 A2**
(43) Date of publication of application: **25.02.2026**
(21) Application number: 25218780.2
(22) Date of filing: 06.06.2023
(51) Int. Cl.: A61F 13/62

(54) **METHODS AND APPARATUSES FOR ALTERING PORTIONS OF SUBSTRATES WITH VIBRATION ENERGY**

(30) Priority: 10.06.2022 US 202263350895 P
(62) Divisional of application: 23177761.6
(71) Applicant: The Procter & Gamble Company, Cincinnati, OH 45202 (US)
(72) Inventor: LENSER, Todd Douglas, Cincinnati, 45202 (US); MYERS, Randall Allen, Cincinnati, 45202 (US)
(74) Representative: P&G Patent Germany

(57) **Abstract**

A method of altering a portion of a substrate may include providing a first device and a second device with a nip therebetween. The second device may comprise a stationary or rotating source of vibration energy. When conveyed through the nip, the substrate may be exposed to the vibration energy and the portion thereof may be altered. Thermal energy may be applied to the portion of the substrate upstream of the nip to raise a temperature of the portion of the substrate to a temperature below a melting temperature thereof. A substrate spreader may contact the substrate upstream of the nip to mitigate fold over or wrinkles in the substrate. When the first device is a rotating anvil and the second device comprises a rotating source of vibration energy, the surface velocities thereof may be variable and may be the same or different.

## Description

### CROSS REFERENCE TO RELATED APPLICATION

This application claims the benefit, under 35 U.S.C. §119(e), of U.S. Provisional Patent Application No. 63/350,895, filed on June 10, 2022, the entire disclosure of which is incorporated herein by reference.

### FIELD

The present disclosure relates generally to altering portions of substrates with vibration energy and more specifically to forming touch fasteners in substrates with ultrasonic energy.

### BACKGROUND

The discussion of shortcomings and needs existing in the field prior to the present disclosure is in no way an admission that such shortcomings and needs were recognized by those skilled in the art prior to the present disclosure.

Sources of vibration energy in combination with an anvil may be used to create bonds in one or more substrates conveyed therebetween and/or to create surface features in one or more substrates conveyed therebetween. Surface features may include projections or hooks, as may be useful in hook and loop fasteners. The sources of vibration energy may use ultrasonic energy. The source of vibration energy may press against the substates to melt, to soften, and/or to deform a portion of the substrates where a bond is desired. To form the projections or hooks, the substrate(s) may be conveyed through a nip between a source of vibration energy and an anvil with a plurality of projection recesses defined therein. The source of vibration energy presses against the substrate to melt, to soften, and/or to deform a portion of the substate into a deformable film that may flow, stretch, and/or otherwise deform into the plurality of projection recesses. The substrate(s) may then be moved out of the nip and the substrate may be removed from the anvil, resulting in a substrate having a plurality of projections or hooks in some regions, melted substrate around the projections or hooks, and/or normal nonwoven or film in other regions (non-melted regions). The plurality of projections may form one side of a touch fastener for an absorbent article.

Existing processes for forming a touch fastener hook patch between a blade-style sonotrode and an anvil roll have multiple failure modes and may only be operated at limited line speeds. Holes, tears, incomplete hook formation, and hard ridges may be observed in the patch, including but not limited to at the trailing edge of the patch. The blade-style sonotrode may have localized high temperature regions, which may burn through a substrate and cause holes or tears. For example, shear stresses in the hot polymer near the stationary sonotrode and the rotating anvil roll may lead to holes in the patch. The hooks formed in the non-woven substrate by the existing processes may have low structural integrity in the region of the base of the hook near the membrane of the substrate and may, therefore, fracture. Existing processes may also be sensitive to the patch shape and the intermittent nature of the patches, which limits product design options. In particular, the shaped patches may require a significant in-nip area balance, which often constrains variation of the cross-directional (CD) width of the patch through the machine-directional (MD) length of the patch. "Area balance" as used herein refers to the area of substrate intermediate the sonotrode and anvil at any instant in time of revolution of the mold roll. A high imbalance may lead to mechanical vibration and uneven forces in the process. Further, process parameters such as applied force, amplitude, and/or frequency of the ultrasonic vibration may be difficult to control throughout a patch with prior art. Shaped and/or intermittent patch shapes and/or higher line speeds may make such process control even more difficult.

A need, therefore, exists for a method and apparatus to create shaped patterns of intermittent touch fasteners in a non-woven at higher line rates without quality defects.

### SUMMARY

Various embodiments solve the above-mentioned problems and provide methods and devices useful for creating shaped patterns of intermittent touch fasteners in a nonwoven at higher line rates without quality defects. Various solutions may include applying thermal energy to a portion of the substrate upstream of a nip formed between a first device comprising an outer surface and providing a second device comprising a source of vibration energy. The thermal energy may be optimized to raise a temperature of the portion of the substrate to a temperature below a melting temperature of the portion of the substrate. To avoid burn-through, the thermal energy may be hindered from reaching the source of vibration energy, other portions of the substrate, and/or the nip. Various solutions may also include optimizing the lineal force, the zero-to-peak sinusoidal amplitude, and/or the frequency of vibration energy applied by the source of vibration energy, particularly a rotating source of vibration energy, like a rotary sonotrode. Wrinkling and other quality defects may be avoided by employing substrate spreaders and by rotating the first device and the second device at different velocities.

More specifically, various embodiments relate to a method of altering a portion of a substrate. The method may comprise providing a first device comprising an outer surface; providing a second device comprising a source of vibration energy; forming a nip between the source of vibration energy and the outer surface; conveying the substrate through the nip; and applying vibration energy from the source of vibration energy to the substrate in the nip to alter the portion of the substrate.

According to various embodiments, the method may also comprise applying thermal energy to a portion of the substrate upstream of the nip to raise a temperature of the portion of the substrate to a temperature below a melting temperature of the portion of the substrate. The temperature below the melting temperature of the portion of the substrate may be in the range of about 90 degrees C to about 160 degrees C. To mitigate fold over or wrinkles in the substrate, particularly in embodiments that apply thermal energy to the portion of the substrate, the method may further comprise conveying the substrate into contact with a substrate spreader upstream of the nip. The thermal energy may have a temperature in the range of about 90 degrees C to about 190 degrees C, about 120 degrees C to about 300 degrees C, or about 120 degrees C to about 190 degrees C.

According to various embodiments, the first device may be a rotating anvil. The second device may be a rotating source of vibration energy, such as a rotary sonotrode or a stationary source of vibration energy, such as a blade sonotrode. When the source of vibration energy is a rotating source of vibration energy, the method may further comprise altering the portion of the substrate in the nip using the rotating source of vibration energy. The rotating source of vibration energy may apply a lineal force of about 1 kN to about 5 kN per 25 mm of operative contact width. The rotating source of vibration energy may have a zero-to-peak sinusoidal amplitude of about 20 to about 60 micron. The rotating source of vibration energy may apply about 15 kHz to about 29 kHz of vibration energy to the substrate. In any case, the source of vibration energy may apply vibration energy continuously or intermittently. The vibration energy may be ultrasonic energy.

When the first device is a rotating anvil and the second device comprises a rotating source of vibration energy, the first device may have a first surface velocity and the second device may have a second surface velocity. The first surface velocity may be variable, and/or the second surface velocity may be variable. Additionally, the first surface velocity and the second surface velocity may be the same or different.

These and other features, aspects, and advantages of various embodiments will become better understood with reference to the following description, figures, and claims.

### BRIEF DESCRIPTION OF THE DRAWINGS

Many aspects of this disclosure can be better understood with reference to the following figures.
Figure 1 is an example according to various embodiments illustrating a perspective view of an apparatus for altering a portion of a substrate comprising a rotating source of vibration energy, upper and lower heat sources, and a substrate spreader.
Figure 2 is an example according to various embodiments illustrating a perspective view of an apparatus for altering a portion of a substrate comprising a rotating source of vibration energy, upper and lower heat sources, top and bottom heat shields, and a substrate spreader.
Figure 3 is an example according to various embodiments illustrating a bottom perspective view of an apparatus for altering a portion of a substrate comprising a rotating source of vibration energy, upper and lower heat sources, top and bottom heat shields, and a substrate spreader.
Figure 4 is an example according to various embodiments illustrating a cross-sectional view of an apparatus for altering a portion of a substrate comprising a rotating source of vibration energy, upper and lower heat sources, top and bottom heat shields, and a substrate spreader.
Figure 5 is an example according to various embodiments illustrating a perspective view of an apparatus for altering a portion of a substrate comprising a stationary source of vibration energy, upper and lower heat sources, top and bottom heat shields, and a substrate spreader.
Figure 6 is an example according to various embodiments illustrating a perspective view of an apparatus for altering a portion of a substrate comprising a stationary source of vibration energy, upper and lower heat sources, top, bottom, and upwardly extending heat shields, a substrate spreader, and a cooling block.
Figure 7 is an example according to various embodiments illustrating a cross-sectional view of an apparatus for altering a portion of a substrate comprising a stationary source of vibration energy, upper and lower heat sources, top, bottom, and upwardly extending heat shields, a substrate spreader, and a cooling block.
Figure 8 is an example according to various embodiments illustrating a perspective view of an apparatus for altering a portion of a substrate comprising a stationary source of vibration energy, upper and lower heat sources, top, bottom, and upwardly extending heat shields, a substrate spreader, and a cooling blower.
Figure 9A is an example according to various embodiments illustrating a side view of a first device comprising a plurality of patterns having recesses or indentations.
Figure 9B is an example according to various embodiments illustrating a cross-sectional view of a recess in one of the patterns.
Figure 10A is a comparative example illustrating a resulting sheet having a plurality of patches, but also a plurality of burnt-through portions.
Figure 10B is an example according to various embodiments illustrating a resulting sheet having a plurality of patches, suitable for use as touch fasteners.
Figure 11 is an example according to various embodiments illustrating a side view of the first device as shown in Figure 9A, including some additional dimensional notations.
Figure 12 is an example according to various embodiments illustrating an end view of a blade sonotrode having a width only slightly wider than the pattern on an anvil roll.

It should be understood that the various embodiments are not limited to the examples illustrated in the figures.

### DETAILED DESCRIPTION

This disclosure is written to describe the invention to a person having ordinary skill in the art, who will understand that this disclosure is not limited to the specific examples or embodiments described. The examples and embodiments are single instances of the invention which will make a much larger scope apparent to the person having ordinary skill in the art. Unless defined otherwise, all technical and scientific terms used herein have the same meaning as commonly understood by the person having ordinary skill in the art. It is also to be understood that the terminology used herein is for the purpose of describing examples and embodiments only, and is not intended to be limiting, since the scope of the present disclosure will be limited only by the appended claims.

All the features disclosed in this specification (including any accompanying claims, abstract, and drawings) may be replaced by alternative features serving the same, equivalent, or similar purpose, unless expressly stated otherwise. Thus, unless expressly stated otherwise, each feature disclosed is one example only of a generic series of equivalent or similar features. The examples and embodiments described herein are for illustrative purposes only and that various modifications or changes in light thereof will be suggested to the person having ordinary skill in the art and are to be included within the spirit and purview of this application. Many variations and modifications may be made to the embodiments of the disclosure without departing substantially from the spirit and principles of the disclosure. All such modifications and variations are intended to be included herein within the scope of this disclosure. For example, unless otherwise indicated, the present disclosure is not limited to particular materials, reagents, reaction materials, manufacturing processes, or the like, as such can vary. It is also to be understood that the terminology used herein is for purposes of describing particular embodiments only and is not intended to be limiting. It is also possible in the present disclosure that steps can be executed in different sequence where this is logically possible.

In everyday usage, indefinite articles (like "a" or "an") precede countable nouns and noncountable nouns almost never take indefinite articles. It must be noted, therefore, that, as used in this specification and in the claims that follow, the singular forms "a," "an," and "the" include plural referents unless the context clearly dictates otherwise. Thus, for example, reference to "a support" includes a plurality of supports. Particularly when a single countable noun is listed as an element in a claim, this specification will generally use a phrase such as "a single." For example, "a single support."

All numeric values are herein assumed to be modified by the term "about," whether or not explicitly indicated. The term "about" generally refers to a range of numbers that one of skill in the art would consider equivalent to the recited value (for example, having the same function or result). In many instances, the term "about" may include numbers that are rounded to the nearest significant figure.

Where a range of values is provided, it is understood that each intervening value, to the tenth of the unit of the lower limit (unless the context clearly dictates otherwise), between the upper and lower limit of that range, and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

"Disposed" is used herein to refer to a positional state indicating that one object or material is arranged in a position adjacent to the position of another object, material, or environment. The term does not require or exclude the presence of intervening objects, materials, or layers.

"Anvil roll" is used herein to refer to any industrial tool comprising an essentially cylindrical form having a pattern or profile on the outer circumferential surface. An anvil roll is on example of a "first device" according to various embodiments. The first device need not be but may be essentially cylindrical. A first device may be any desirable shape, including but not limited to concave, convex, or flat according to various embodiments.

"Absorbent article" is used herein to refer to consumer products whose primary function is to absorb and retain soils and wastes. "Diaper" is used herein to refer to an absorbent article generally worn by infants, children, and incontinent persons about the lower torso. "Diaper" may include taped diapers and pant-type diapers. The term "disposable" is used herein to describe absorbent articles which generally are not intended to be laundered or otherwise restored or reused as an absorbent article (e.g., they are intended to be discarded after a single use and may also be configured to be recycled, composted, or otherwise disposed of in an environmentally compatible manner).

"Elastic," "elastomer" or "elastomeric" are used herein to refer to materials exhibiting elastic properties, which include any material that upon application of a force to its relaxed, initial length can stretch or elongate to an elongated length more than 10% greater than its initial length and will substantially recover back to about its initial length upon release of the applied force.

"Substrate" is used herein to describe a material which is primarily two-dimensional (i.e., in an XY plane) and whose thickness (in a Z direction) is relatively small (i.e. 1/10 or less) in comparison to its length (in an X direction) and width (in a Y direction). Non-limiting examples of substrates include a web, layer or layers or fibrous materials, nonwovens, films, and foils such as polymeric films or metallic foils. These materials may be used alone or may comprise two or more layers laminated together. As such, a web is a substrate.

"Nonwoven" is used herein to refer to a material made from continuous (long) filaments (fibers) and/or discontinuous (short) filaments (fibers) by processes such as spunbonding, meltblowing, carding, and the like. Nonwovens do not have a woven or knitted filament pattern.

"Machine direction" (MD) is used herein to refer to the direction of material flow through a process. In addition, relative placement and movement of material can be described as flowing in the machine direction through a process from upstream in the process to downstream in the process.

"Cross direction" (CD) is used herein to refer to a direction that is generally perpendicular to the machine direction.

"Sonotrode" refers to an ultrasonic machining, welding, and/or mixing tool that creates ultrasonic vibrations and applies this vibrational energy to a gas, liquid, solid or tissue. A sonotrode may have a variety of shapes and may be stationary or may rotate. The terms sonotrode and ultrasonic horn may be one and the same, and thus may be used interchangeably.

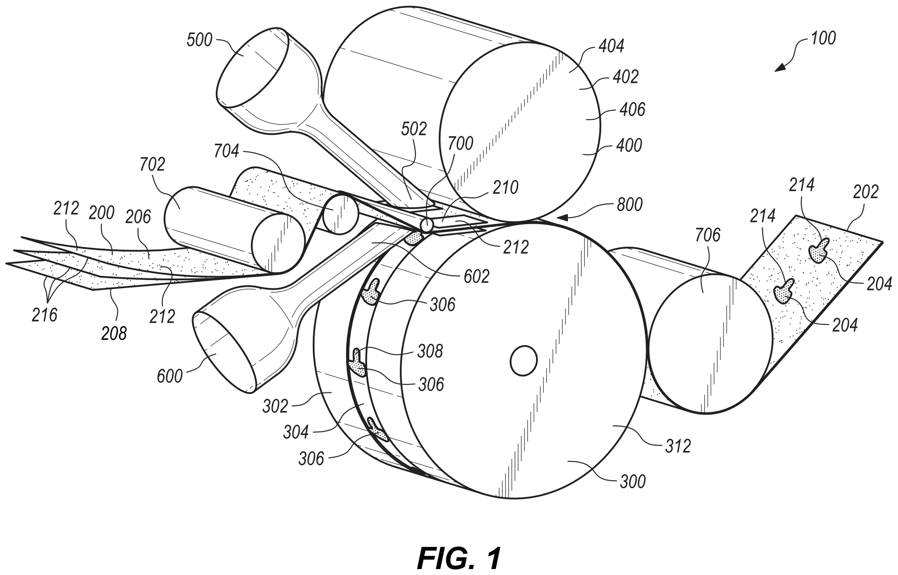

To provide an overview, Figures 1 - 8, 9A, 9B, 10A, and 10B will be referenced generally; individual figures and groups of figures are discussed hereinafter. Various embodiments relate to a method of altering a portion of a substrate 200. It is to be appreciated that the methods of the present disclosure may also be used to create ultrasonic bonds for laminates, such as back ear laminates, waistbands, and waistcuffs for absorbent articles. The substrate 200 may comprises one layer or more than one layer 216. The substrate 200 may comprise one material or more than one material. The substrate 200 may be, but need not be limited to, a film, sheet, web, nonwoven, composite, laminate, or other form, or may be portions of a film, sheet, web, nonwoven, laminate or substrate thermoplastic material, portions of which may be used as a component of a touch fastener, for instance on a absorbent article. In their use on absorbent article, touch fasteners may be attached to a "side tab" or "ear" that the consumer uses to secure the absorbent article the wearer. These tabs may be constructed with a piece of extensible material to allow the side tab to stretch and flex when attached or when the wearer moves. The touch fasteners may also be used in a two-point fastening system on an absorbent article, where the component is positioned on a landing zone or outer cover of the absorbent article. The present disclosure further contemplates the use of pre-formed film, sheet, web, composite, laminate, etc. as a substrate material.

Any of the substrates described herein may comprise one or more layers 216 of one or more nonwoven materials, one or more films, combinations of different nonwoven materials, combinations of different films, combinations of one or more films and one or more nonwoven materials, or combinations of one or more different materials, for example, depending on the specific purpose for which they are intended. The substates may comprise spunbond, meltblown, or carded materials. The substrates may also comprise polyethylene films. The nonwoven substates may or may not be hydroentangled.

Some substrates for nonwoven materials may comprise PE/PP bicomponent fiber spunbond substates. Other suitable substrates may comprise spunbond substrates comprising side-by-side crimped fibers (e.g., PE/PP or PP/PP) that are bonded via calendar (thermal point) bonding or through-air bonding. Other suitable substrates may comprise carded, through-air bonded or resin bonded (highloft) nonwovens comprising PE/PP or PE/PET fibers. The substates may comprise micro fibers and/or nanofibers, optionally with other fibers. In some circumstances, multiple layer substates may be desired over a single layer substates (even at the same basis weight) due to increased uniformity/opacity and the ability to combine substrates having different properties. The layers may have the same or different surface energy.

Fibers of the substrates may comprise any suitable thermoplastic polymers. Example thermoplastic polymers are polymers that melt and then, upon cooling, crystallize or harden, but that may be re-melted upon further heating. Suitable thermoplastic polymers may have a melting temperature (also referred to as solidification temperature) from about 60°C to about 300°C, from about 80°C to about 250°C, or from about 100°C to about 215°C, specifically reciting all 0.5°C increments within the specified ranges and all ranges formed therein or thereby. And the molecular weight of the thermoplastic polymer may be sufficiently high to enable entanglement between polymer molecules and yet low enough to be melt spinnable.

The thermoplastic polymers may be derived from any suitable material including renewable resources (including bio-based and recycled materials), fossil minerals and oils, and/or biodegradable materials. Some suitable examples of thermoplastic polymers include polyolefins, polyesters, polyamides, copolymers thereof, and combinations thereof. Some example polyolefins include polyethylene or copolymers thereof, including low density, high density, linear low density, or ultra-low density polyethylenes.

The thermoplastic polymer component may be a single polymer species or a blend of two or more thermoplastic polymers e.g., two different polypropylene resins. As an example, fibers of a first nonwoven layer of a substrate may comprise polymers such as polypropylene and blends of polypropylene and polyethylene, while a second nonwoven layer of the substate may comprise fibers selected from polypropylene, polypropylene/polyethylene blends, and polyethylene/polyethylene terephthalate blends. In some forms, the second nonwoven layer may comprise fibers selected from cellulose rayon, cotton, other hydrophilic fiber materials, or combinations thereof. In some forms, the plurality of substrate polymers or component fibers may have different melting temperatures, such as by at least about 10°C or at least about 30°C, up to about 100°C, specifically reciting all 1°C increments therebetween. In other embodiments, one of the components fibers, such as a cellulose-based component, may not have a melt temperature. A combination of a higher melt temperature polymer for structural integrity with a lower melt temperature polymer to soften and form bonds between adjacent fibers may be employed.

The fibers of the layer of the substrate may comprise monocomponent fibers, bi-component fibers, and/or bi-constituent fibers, round fibers, or non-round fibers (e.g., capillary channel fibers), and may have major cross-sectional dimensions (e.g., diameter for round fibers) ranging from about 0.1 microns to about 500 microns. The fibers may also be a mixture of different fiber types, differing in such features as chemistry (e.g., polyethylene and polypropylene), components (mono- and bi-), denier (micro denier and >2 denier), shape (i.e. capillary and round) and the like. The fibers may range from about 0.1 denier to about 100 denier.

Example substrates are contemplated where a first plurality of fibers and/or a second plurality of fibers comprise additives in addition to their constituent chemistry. For example, suitable additives include additives for coloration, antistatic properties, lubrication, softness, hydrophilicity, hydrophobicity, and the like, and combinations thereof.

As used herein, the term "monocomponent fiber(s)" refers to a fiber formed from one extruder using one or more polymers. This is not meant to exclude fibers formed from one polymer to which small amounts of additives have been added for coloration, antistatic properties, lubrication, hydrophilicity, etc.

As used herein, the term "bi-component fiber(s)" refers to fibers which have been formed from at least two different polymers extruded from separate extruders but spun together to form one fiber. Bi-component fibers are also sometimes referred to as conjugate fibers or multicomponent fibers. The polymers are arranged in substantially constantly positioned distinct zones across the cross-section of the bi-component fibers and extend continuously along the length of the bi-component fibers. The configuration of such a bi-component fiber may be, for example, a sheath/core arrangement where one polymer is surrounded by another or may be a side-by-side arrangement, eccentric arrangements, a pie arrangement, or an "islands-in-the-sea" arrangement. Some specific examples of fibers which may be used in the first nonwoven layer include polyethylene/polypropylene side-by-side bi-component fibers. Another example is a polypropylene/polyethylene bi-component fiber where the polyethylene is configured as a sheath and the polypropylene is configured as a core within the sheath. Still another example is a polypropylene/polypropylene bi-component fiber where two different propylene polymers are configured in a side-by-side configuration. Additionally, forms are contemplated where the fibers of a nonwoven layer are crimped.

Bi-component fibers may comprise two different resins, e.g., a first polypropylene resin and a second polypropylene resin. The resins may have different melt flow rates, molecular weights, or molecular weight distributions, or melting properties.

As used herein, the term "bi-constituent fiber(s)" refers to fibers which have been formed from at least two polymers extruded from the same extruder as a blend. Bi-constituent fibers do not have the various polymer components arranged in relatively constantly positioned distinct zones across the cross-sectional area of the fiber and the various polymers are usually not continuous along the entire length of the fiber, instead usually forming fibrils which start and end at random. Bi-constituent fibers are sometimes also referred to as multi-constituent fibers. In other examples, a bi-component fiber may comprise multiconstituent components.

As used herein, the term "non-round fiber(s)" describes fibers having a non-round cross-section and includes "shaped fibers" and "capillary channel fibers." Such fibers may be solid or hollow, and they may be tri-lobal, delta-shaped, and may be fibers having capillary channels on their outer surfaces.

Other example nonwoven materials for the substates may comprise spunlace materials, needle punched materials, wet-laid materials, or air-laid materials, for example.

Still referring to Figures 1 - 8, 9A, 9B, 10A, and 10B generally, the method of altering a portion of a substrate 200 may comprise providing a first device 300 and a second device. The first device may comprise an outer surface 302. The first device 300 may be an anvil roll 308 having one or more patterns 306 on its outer surface 306 or on an indented portion 304 of its outer surface 306. For example, the method may comprise providing a plurality of recesses 308 in the outer surface 302 of the first device 300. The recesses 308 may have a shape 310 configured to produce projections 214 suitable for use in a touch fastener.

The second device 400 may comprise a source of vibration energy 402. The source of vibration energy 402 may be a stationary source of vibration energy 410, such as a blade sonotrode 408. Alternatively, the source of vibration energy 402 may be a rotating source of vibration energy 404, such as a rotary sonotrode 406. The source of vibration energy 402 may apply vibration energy to the substrate 200 intermittently or continuously. The vibration energy may be ultrasonic energy.

The method may further comprise forming a nip 800 between the source of vibration energy 402 and the outer surface 302 of the first device 300 and conveying the substrate 200 through the nip 800. As the substrate 200 is conveyed through the nip, the method may comprise applying vibration energy from the source of vibration energy 402 to the substrate 200 in the nip 800 to alter the portion 210 of the substrate 200. Altering the substrate may include forming bonds, three-dimensional features, projections, and/or hooks, for example.

According to various embodiments it has been discovered that operating a rotating source of vibration energy, such as a rotary sonotrode, under specific conditions produces beneficial results for producing patches comprising hook protrusions for touch fasteners. Relative to the blade sonotrode, the rotary sonotrode may deliver slower throughput, but it was discovered that when operated under specific conditions, which are detailed herein, the patches comprising hook protrusions for touch fasteners produced by the rotary sonotrode exhibited better quality. These results were surprising, because blade sonotrodes have a higher potential zero-to-peak amplitude, as high as ~45 microns. It was discovered, however, that blade sonotrodes must frequently be limited to a zero-to-peak amplitude of 25 microns or less in processes for producing patches comprising hook protrusions for touch fasteners to avoid hole formation in the substrate.

Embodiments employing a rotating source of vibration energy, such as a rotary sonotrode, and specific operating parameters may enable a wider variety of hook patch shapes to be produced, reduce quality defects, such as holes or tears in the substrate, may reduce distortions at the trailing edge of a patch, may reduce shear stresses on the substrate at the nip, and/or may reduce localized high temperature regions on the substrate and/or on the sonotrode compared to those observed on blade sonotrodes. Moreover, the patches produced by the rotary sonotrode, operated under specific conditions, exhibited higher bond forces when used as touch fasteners in product shear tests and a more uniform hook field with higher hook counts. Without being limited by theory, it is believed that under certain operating conditions, the rotary sonotrode creates a melt pool at the infeed to the nip, which may enable cross-direction CD and machine direction MD flow of heated substrate material to feed hook cavities and to mitigate the natural variations in localized non-woven basis weight. Without being limited by theory, it is believed that the higher contact pressures of the rotary sonotrode enhance the bonding of adjacent fibers within hooks and/or the structural integrity of the hook to the membrane at the hook to membrane interface. The large surface area, high mass, and limited duty cycle of any localized region of the rotary sonotrode may also enable a lower and more consistent temperature compared to a blade sonotrode, which may reduce burn through.

The parameters employed according to various embodiments are outside those common in the industry for rotary ultrasonic bonding. It was further discovered not all rotary ultrasonic systems are suitable for formation of secondary hook fasteners, primary hook fasteners, and/or disposal tapes. For example, it was discovered that 30kHz and 35kHz rotary ultrasonics systems were only capable of about 1500N force, and a power output of ~600w and 1,600W respectively, with a narrow working width and lower amplitude. Such rotary sonotrodes do not have sufficient compression force, precision alignment, amplitude, or power capacity for producing patches comprising hook protrusions for touch fasteners.

Generally, various embodiments operate at high compressive force, low material speed, and high ultrasonic amplitude. Without being limited by theory, it is hypothesized that in addition to increasing overall energy, the higher amplitude increases the apparent shear rate and thereby decreases the apparent viscosity, elastic modulus and in the case of a fibrous material, the yield point of the polymer based fibers, which may improve cavity packout. "Packout" as used herein refers to the filling of the cavity with polymer. As a non-limiting example, a 20kHz, 85mm CD width rotary sonotrode (ultrasonic sonotrode), which featured bearings on both operator and drive sides of the sonotrode was tested. The bearing arrangement and stiff mounting bracket enables a precise alignment between the rotary sonotrode and the anvil roll with minimal deflection compared to cantilevered rotary ultrasonics systems. Precision alignment is required due to the thin 20 - 25 micron membrane typical of unitary touch fasteners. This system was capable of up to 5000 N compression force, which substantially improved the flow of polymer into the plurality of cavities on the anvil roll. Hook formation improved with setpoints of 2.8 - 3.5kN for a 20 - 36mm wide shaped pattern, with even higher forces potentially desired. The highest amplitude settings, roughly 22 microns zero-to-peak amplitude improved flow of the polymer into the hook cavities.

In processes for producing patches comprising hook protrusions, it has been discovered that rotary sonotrodes operated under certain conditions may mitigate localized regions of increased force and/or stress at leading and trailing edges of the patch. Either rotary or blade sonotrode may nominally operate in a position control mode, such as via a stepper motor positioner, precise to within 0.3 micron. A force sensor may be used in closed-loop feedback control to measure the reaction force from compressing the raw material stack with the sonotrode. The closed-loop controller may adjust the position of the sonotrode to decrease or increase a distance between the sonotrode and the periphery of the anvil roll. The stepper position control may operate at a much longer time than the duration of a patch. A higher force generally corresponds to a thinner membrane intermediate the formed hooks. A thinner membrane region may be expected to tear more easily. The sonotrode stack may not be infinitely stiff, and may act to some extent as a compression spring under the relatively high reaction forces (e.g. 2,500 - 4,000N per 20-36mm CD width) favorable to the process. Preloading the sonotrode mounting system against stiff spring elements, including such that the system is in compression throughout the cycle to mitigate backlash, may reduce the effects. At the leading edge of a patch, the stack may compress slightly, even by microns, as the leading edge of the patch on the anvil roll rotates under the sonotrode. On a blade sonotrode, this may lead to a time transient, localized region with higher compression force and/or a thinner membrane region. Until the anvil roll patch is fully under the sonotrode, this force may be applied over a smaller than steady state area, resulting in high pressure relative to a central region of the patch. While hooks may be well-formed, the membrane area may be thinned due to the high pressure in this localized region. The thin membrane may have an increased propensity to tear, resulting in product holes. There may also be an amplitude peak overshoot and oscillation relative to setpoint due to the dynamics of the closed feedback loop amplitude control of the sonotrode. The localized higher amplitude may squeeze more polymer out of a localized region to create thinner regions. Further, the leading edge of the patch may have a high shear force. Particularly with a blade sonotrode, the upstream edge of the sonotrode may induce a shear force across the material at the leading edge of the anvil roll pattern. With a rotary sonotrode, these failure modes may be mitigated by the cylindrical nature of the sonotrode. The rotary sonotrode may generate a lower or no shear force due to a stress concentration at the leading edge, as it is rotating with the substrate. The rotary sonotrode may compress the substrate, which may be 200 microns or more thick, to 20 - 40 microns before squeezing some of the polymer into the anvil roll cavities. This compression may be substantially due to the in-running nip formed between the two sonotrodes, rather than solely due to sonotrode geometry as occurs on a blade sonotrode.

At the trailing edge of the patch, the effect may be similar and may occur even more often. The sonotrode stack may be compressed through the majority of the patch, with a preloaded force. As the patch rotates out of the nip, the in-nip area of patch surface under the sonotrode will decrease. The compressive pressure will increase as force remains similar, but area is reduced. This high compressive pressure tends to create a thin membrane, which may be more susceptible to shearing. The shear stress may also be increased by the locally high compression stress. In the case of a blade sonotrode, this effect may be worse due to a molten or semi-molten polymer accumulation upstream the sonotrode. The accumulated melt pool may help feed the cavities but may deposit at the trailing edge of the patch. The melt pool is commonly observed via high-speed infrared video of the process, where it appears as a hot line of ~0.5 - 2mm MD length along the trailing edge of the non-woven. Once this region of polymer cools, it may form a ridge, which may be hard and objectional on an aesthetic basis to the end user. The melt pool may remain at an elevated temperature, with lower elastic modulus and/or remaining semi-molten, during, through, or until the process of hook extraction from the mold. The melt pool or the thin region adjacent may tear under substrate tension as the hooks are stripped from the anvil roll. In product tests, the rotary system substantially reduced the incidence of hard trailing edge ridges and holes adjacent the trailing edge of the patch.

The thinner trailing edge may result in reduced gauge bands in the finished roll, particularly compared to blade sonotrodes, which may form a thicker region around the hook patch. Upon rewind into a finished roll of non-woven, several hundred or even a few thousand layers of the patches may stack in layers. These thicker regions may lead to an uneven radius, especially across the CD, in the finished roll, with thicker or higher radius regions referred to as gauge bands associated with the hook patches. Gauge bands distort during both winding and unwinding, and may form wrinkles, foldovers, holes, and/or cause the substrate to mistrack during processing. The wrinkles may interfere with quality measurements, phasing or MD registration controls, CD tracking, or placement of a cut landing zone or other product element upon a diaper. Where wrinkles, foldovers, or deformations in the patch exist, the finished product may be defective, have reduced function, or be aesthetically unpleasing. Rotary ultrasonics reduced these defects.

According to various embodiments, it has been discovered that operating a rotating source of vibration energy 404 within certain parameters yields beneficial results. For example, beneficial results may be obtained when the rotating source of vibration energy 404 applies a lineal force of about 1 kN to about 5 kN, or about 2 kN to about 4 kN, or about 3 kN to about 5 kN, or about 1 to about 3 kN per 25 mm of operative contact width. Similarly, beneficial results may be obtained when the rotating source of vibration energy 404 has a zero-to-peak sinusoidal amplitude of about 20 to about 60 microns, or about 25 to about 55 microns, or about 30 to about 50 microns, or about 35 to about 45 microns, or about 40 to about 60 microns, or about 20 to about 40 microns. Finally, beneficial results may be obtained when the rotating source of vibration energy 404 applies about vibration energy to the substrate at a frequency of about 15 kHz to about 29 kHz, or about 20 to about 25 kHz. All frequencies provided are +/-200Hz or +/-500Hz.

According to various embodiments, it has also been discovered that operating a rotating source of vibration energy 404, such as a rotary sonotrode 406 in conjunction with a rotating first device, such as a rotating anvil roll, within certain parameters yields beneficial results. For example, when the first device is a rotating anvil and the second device comprises a rotating source of vibration energy, the first device may have a first surface velocity 314 and the second device may have a second surface velocity 412. The first surface velocity 314 may be variable, and/or the second surface velocity 412 may be variable. Additionally, the first surface velocity 314 and the second surface velocity 412 may be the same or different.

It has been discovered that causing a speed mismatch between the motor-driven rotary sonotrode and the anvil roll may improve hook formation for some process settings. According to various embodiments, a speed mismatch applied to the rotary system may improve packout of polymer into the recesses, thereby improving hook formation and the resultant hook structure.

"Overspeed" as used herein refers to an operating condition in which the second surface velocity 412 of the second device 400 is higher than the first surface velocity 314 of the first device 300. An overspeed of 5% means that the second surface velocity 412 is 5% greater than the first surface velocity 314. Without being limited by theory, it is hypothesized that the overspeed draws material into the nip and/or increases the shear rate, which may lower the apparent viscosity of the heated polymer substrate to improve polymer flow. According to various embodiments an overspeed of about 5%, about 10%, about 15%, about 20%, about 25%, about 30 %, about 40%, about 45%, about 50%, about 55%, about 65%, about 70%, about 75%, about 80%, about 85%, about 90%, about 95%, or up to about 100% may be employed.

"Underspeed" as used herein refers to an operating condition in which the second surface velocity 412 of the second device 400 is lower than the first surface velocity 314 of the first device 300. An underspeed of 5% means that the second surface velocity 412 is 5% less than the first surface velocity 314. Without being bound by theory, it is hypothesized that underspeed enables the rotary sonotrode to squeeze material upstream, i.e. out of the nip, providing a small molten pool of polypropylene at the upstream edge of the nip point. This melt pool is believed to smear the melt in the cross direction CD, which may average out the basis weight of polymer across the substrate. Initial attempts to run the rotary sonotrode in an "underspeed" configuration failed, causing wrinkles, foldovers, and a slack substrate. For example, the substrate may become slack, or have little or no tension in localized regions just upstream of the nip between the sonotrode and anvil roll. This low tension may interact with shear stresses in the non-woven sheet, which may lead to compressive principal stresses in the cross-machine direction. The shear stresses may be caused by any of non-uniform roll geometry in a cross-machine CD direction or machine direction MD, localized basis weight and/or modulus variation in the non-woven, uneven temperature distribution, spreading mechanism such as flat or bow bars, impinging air jets, misalignment of components, contact forces between sonotrode and non-woven, or a number of other root causes. Significant wrinkling of the substrate with the slack substrate conditions may also be observed due to the compressive principal stresses exceeding the planar buckling critical stress of the raw material. Such wrinkles may substantially negatively impact hook formation, resulting in foldovers and holes in the finished material.

According to various embodiments substrate handling modifications may enable underspeed operation of the rotary sonotrode without causing wrinkles. The substrate handling changes, which are described more fully later, may comprise a lower metering rate to increase substrate tension upstream of the sonotrode, a spreading mechanism, such as a round bar, close to the sonotrode, a heating mechanism close to the sonotrode, and one or more idlers to provide lateral stability and localized moment support to the substrate. According to various embodiments an underspeed of about 5%, about 10%, about 15%, about 20%, about 25%, about 30%, about 35%, or up to about 40% may be employed. The underspeed may improve pack out of the cavities in the anvil roll with polymer.

With overspeed or underspeed, a relatively consistent shear stress may be applied via the rotary anvil to the in-nip polymer, in superposition with the compressive stresses from compression via the vibrating sonotrode. This shear stress may be smaller and/or more uniform than localized high shear stresses, for example, such as with a blade sonotrode. The higher shear rate may reduce the apparent viscosity, improving flow. There may also be an accumulation of polymer at the upstream cusp of the nip region. This accumulation of material may flow in the MD and/or CD to fill cavities. The rotary in-running nip may accomplish this packout and flow despite a shorter nip area, and the contact region of the rotary system may be reduced by about 25% to about 75% compared to the blade system. As the force settings are similar, but the rotary may have reduced in-nip material, the stresses in the in-nip polymer of the rotary system causing flow are predicted to be higher. The rotary system may deform the material into the cavities due to higher forces, even if the ultrasonic energy addition is lower than via a blade sonotrode. The material formed may appear different and the finished product may have increased opacity and/or reduced glossiness. This appearance may be due to more intact fibrous structures, with diffraction at boundaries between fibers. In some instances, this increased opacity or reduced gloss may be beneficial. With underspeed, matched speed, or overspeed of a rotary sonotrode to the anvil roll surface velocity, the boundaries of the patch are well formed and soft, with no holes or hard ridges.

It has further been discovered that pre-heating a specific, limited portion of the substrate yields beneficial results. For example, according to various embodiments, the method may comprise imparting thermal energy to a portion 210 of the substrate 200 upstream of the nip 800 to heat the portion 210 of the substrate 200 to a temperature below a melting temperature of the portion 210 of the substrate 200. The temperature below the melting temperature of the portion 210 of the substrate 200 may be in a range of about 90 degrees C to about 160 degrees C, or about 100 degrees C to about 150 degrees C, or about 110 degrees C to about 140 degrees C, or about 120 degrees C to about 130 degrees C, or about 90 to about 125 degrees C, or about 125 degrees C to about 160 degrees C. According to various embodiments, the thermal energy may be applied to a first side 206 of the substrate 200 and/or to a second side 208 of the substrate 200. The method may further comprise maintaining the temperature below the melting temperature of the portion 210 of the substrate 200 until the portion 210 of the substrate 200 is conveyed through the nip 800.

It has been discovered that rotary sources of vibration energy, such as rotary sonotrodes typically provide a lower amplitude than a stationary source of vibration energy, such as a blade sonotrode. Rotary sonotrodes may have a shorter contact time for a given line speed, which may reduce the energy transfer for a given machine rate relative to blade style sonotrodes. To compensate, various embodiments may pre-heat the non-woven to a temperature slightly less than its melt temperature (or glass transition temperature). Preheating may enable both higher quality and higher line speeds. Heat may be applied from either or both of the anvil roll side and/or sonotrode side of the material. A suitable heating range for many embodiments may be in a range of about 95°C to about 110°C. Higher temperatures may tend to cause holes in the non-woven near the patches.

Pre-heating the substrate in combination with mis-matched speeds may significantly enhance the process and may enable high quality hook patches at or more than double line speed compared to a process without pre-heat. Pre-heat may help to achieve a high-quality hook at elevated speeds, with significant sensitivity to the actual non-woven preheating temperature. Preheating may be accomplished via convective heat transfer from jets of air at elevated temperature. Pre-heating may also be effective with blade sonotrodes. Pre-heating may be performed from a first side of the substrate, on a second side of the substrate, or on both sides of the substrate. Pre-heating may also be performed when the substrate is wrapped on the anvil roll, including with about 90° of wrap angle on the anvil roll, with either the first side or the second side of the substrate directed at the anvil roll rather than the non-woven.

Preheating may be via hot air and/or radiative heat transfer, including but not limited to infrared heaters, lasers, or radio-frequency heating. Preheating may be via conductive heat transfer such as one or more heated roller(s), for example. The pre-heating via conduction may be similar to pattern rolls and anvils known in the non-woven making art as primary thermal bonds, used to consolidate a batt of non-woven fiber. A region substantially including the patch region may be partially or fully pre-consolidated by compression with a heated machine element prior to application of ultrasonics. Multiple pre-heat methods may be used in series. For example, a mechanical heated compression roll may be used for initial consolidation, followed by convective heating and an ultrasonic sonotrode. Patterned preheating of convective, radiative, or conductive methods may be intermittent, shaped, and/or phased and disposed in the cross-direction CD to correspond to the region where unitary protrusions from the material are desired.

It was discovered that heating was effective when the substrate was disposed with a nominally tangent entry to the nip point intermediate anvil roll and sonotrode. A slight wrap of the substrate on anvil roll of about 10 - 30mm was useful to stabilize the substrate. The heating may be to an indicated substrate pre-heat temperature of about 95°C to 110°C for a polypropylene nonwoven, for example. According to other embodiments, the pre-heat may be as high as 130 - 135°C on the substrate. The temperature will vary based on the properties of the particular substrate, different polymers having different melting temperatures. Melting temperature for a polypropylene nonwoven may be about 160°C or initiate at ~148°C and complete by 165°C for a typical isotatic polypropylene. The pre-heat temperature may be near the melting temperature and may result in a portion of some substrate fibers partially melting. Temperature measurements may be obtained by any suitable methods, such as via an infrared camera, or an infrared meter. The devices require an accurate emissivity coefficient, which may not be readily available for the substrate. According to various embodiments, the pre-heat temperature may be as close to the melt temperature as practicable, subject to process and product constraints which may include burn-through, holes, wrinkling, hard spots, a rough tactile feel, or other undesirable product and/or process effects.

The substrate materials used in disposable absorbent articles may often be low basis weight, or low mass per area. As such, a small addition of thermal energy may significantly raise the temperature of the substrate. Likewise, the material may cool quickly when heat is removed. An example embodiment may be 45 gram/m^2 (gsm) polypropylene. For the example at 50 m/min speed, a 20mm width, and a temperature rise from 27°C to 162°C, only about 190 watts per protrusion lane may theoretically be required for the sensible heat portion of the temperature rise. The local material basis weight of a substrate may vary, even when the average value is well controlled. A characteristic dimension of the variation may be 0.5-10mm. At high basis weight regions, the substrate may not be sufficiently heated, and hooks may not sufficiently form. At low basis weight regions, the temperature rise may be excessive, and the material may melt and/or burn through, resulting in holes. The substrate, or structure of fibers, may locally contract due to shrinkage as the fibers are heated. The fibers within primary bonds in the precursor substrate may contract less. Contraction and/or melting of fibers may result in wrinkling of the substrate and or holes in the substrate. A practical method of limited burn-through is to control the temperature of the impinging air stream. The air stream may be deliberately close to the desired pre-heat temperature of the substrate, to avoid excessive heating. The air stream may be disposed to continue from the heating region to the sonotrode, which may reduce cooling intermediate pre-heating and application of ultrasonic energy. For example, the temperature of the air stream may be about 135° to about 162°C for a desired pre-heated substrate temperature of 100-105°. For example, the peak substrate temperature in the pre-heat region may be 105-115°C for a temperature adjacent the sonotrode of 95-110°C. While higher temperature air streams, such as 260° C are readily achievable, burn through may be near instantaneous and difficult to control.

The pre-heat temperature may be controlled dynamically as a function of line speed, which may use a lower temperature at thread or ramp up, and higher temperature as line speed increases. A commercial closed loop temperature controller may be utilized. A thermocouple may be disposed in the heated air stream, and used as a sensor input for manual control, closed loop control, and/or quality monitoring. The thermal couple may be a type K miniature thermocouple, disposed in a central region of the heater nozzle. A non-contact IR sensor, such as those made by EXERGEN^{™}, or an IR camera may be used to monitor the temperature of the pre-heated material at combining. The volumetric air flow may be constant or dynamic. The volumetric air flow may be increased with line speed. The size of the heat transfer region with an impinging jet upon the substrate may be increased for higher line speed, such as by heating the material with a longer machine direction MD distance as line speed is increased. To mitigate cooling between heater and ultrasonic forming, the heating jet may be disposed as close to the sonotrode as practicable.

Without being limited by theory, it is hypothesized that the elevated temperature may also change the substrate's polymer structure and/or macro structure versus ultrasonic formation of unitary touch fasteners without heat. The higher in-process temperature may alter amount and/or rate of polymer crystallization in the cooling process post-formation. The modulus of the hook polymer may be altered from the original material, or from the modulus of the substrate undergoing the process without pre-heating. The in-process Young's modulus and yield stress may be substantially lowered by pre-heating, which may affect flow into cavities and/or extraction from cavities. The in-process apparent viscosity of the polymer may change significantly due to the pre-heat. The outer sheath of the substrate fibers may partially melt. The elevated temperature may increase diffusion rates between adjacent fibers, which may increase bond strength between adjacent fibers. Increased bond strength between fibers may result in a higher material strength and reduced fracture of hooks during extraction from the anvil roll. Increased bond strength between fibers may result in a stiffer structure, which may provide higher shear or peel strengths in a disposable absorbent article finished product. Increased diffusion and bonding between fibers may change the appearance of the membrane region of the patch intermediate protrusions, which may decrease diffraction at fiber boundaries and may change the opacity, including increasing the transparency of the membrane region.

Pre-heating may benefit either rotary or blade sonotrode embodiments of the ultrasonically formed unitary touch fasteners. As previously discussed, the fibers may more fully entangle and/or melt together. Mechanical entanglement due to post yield deformation or due to melting and fusing are not easy and likely not possible to distinguish. Also, the two regimes may appear similar under a scanning electron microscope (SEM). The resulting membrane region may have reduced diffraction between fibers and may appear more transparent and/or reflective. Product graphics under the transparent membrane, which may be used as an insignia to highlight the product feature, may be more obvious to the customer. Improved flow into the anvil roll cavities may consume more of the polymer, so less polymer accumulates upstream the sonotrode and is deposited as a hard ridge at the trailing edge of the hook patch. Due to the reduced in-nip time of the rotary ultrasonics, and therefore limited energy transfer via ultrasonics, the pre-heating is particularly important and efficacious for rotary, enabling an increase about 200% with similar product quality. The blade embodiment is also greatly improved, with an increase up to about 200%.

At 30 m/min and above with a rotary sonotrode, it was discovered that preheating the material may be critical to successful converting of certain materials. For example, a 45gsm polypropylene substrate landing zone, a substrate temperature immediately before combining of about 95-109°C may be employed. Preheating to a lower temperature of 75°C, 85°C, or 90°C may be less effective. Higher temperatures result in melting of the substrate, with burn through, holes, and deformation of the finished product in unintended locations. Temperature measurements were based on an infrared high speed camera, and may be offset from actual temperatures. Regardless of any substrate temperature measurement errors, it was discovered that a hot air jet with a temperature of 275°F (135°C) to 350°F (177°C) worked the best, with higher temperatures for higher line speeds. According to various embodiments, therefore, the preheating may be performed by impinging one or more air streams on the substrate prior to its entry into the nip. The air streams may have a temperature of from about 130°C to about 200°C, or about 140°C to about 190°C, or about 150°C to about 180°C, or about 160°C to about 170°C. The air temperatures may be measured via a thermocouple in the air jet. Depending on the particular polymer, the structure may be amorphous or semi-crystalline. The material may contract when heated, particularly in the fiber longitudinal direction, which is the substrate CD direction for our NWLZ with anisotropic fiber laydown and nominally CD aligned thermal bond pattern.

According to various embodiments, heating via convective heat transfer from a hot air jet impinging against the substrate from both the top and bottom was tested. Nozzles were nominally round tubes of 1" ID for a 20mm CD wide patch, with a typical air velocity of 10-30m/s or more preferably about 20-24m/s. The shape of the tubes is not critical. Air temperature was typically 275°F to about 375°F (135°C to ~165°C). Air temperature was quantified via a type K thermocouple inserted in a central region of the hot air stream. Heating was via a 1740 W resistive heater element, one in each of the top and bottom heaters. Commercial hot air guns where used, comprising a centrifugal blower and resistive heating element. The round tubes are cut at a 20°-45° angle and adjusted close to the nip point between rotary sonotrode and anvil. Later, rubber and/or paper tips were added to the hot air nozzles. Soft tip materials are preferred, to prevent damage to anvil roll or sonotrode if the nozzles should accidently contact the expensive tooling. An S-wrap configuration of counterclockwise and clockwise wrapped idlers provides support for the incoming substrate. The last control point is about 90mm, or as close as practicable, from the nip point between anvil roll and sonotrode. While heating the substrate with an external hot air blast while it wraps the anvil is effective, it was discovered that best results with a NW substrate path which is nominally tangent to both sonotrode and anvil roll, enabling heating as close to the nip point as practical. Nozzles may be as close as 0.5-1.0mm from the anvil roll, sonotrode, and/or a heat shield adjacent the sonotrode. Nozzles may be 0.5 -15mm from the substrate. The heat affected zone may be within 50mm or less of the nip point. The round nozzles are a simple first embodiment. More effective results with lower hot air temperatures were demonstrated with a shaped rubber nozzle, which provided a semi-rectangular air jet cross-sectional shape. The rubber may more preferably be a silicone or other relatively soft material, to reduce the risk of sonotrode or anvil roll damage in case of collision, such as may happen during a jam or wrap of the machinery. Alternately, results were demonstrated with simple paper nozzles. The airflow may be directed primarily in the MD and at a shallow angle relative to the substrate, such that the air jet continues to the sonotrode and the substrate is protected from cooling. The flow from the nozzles may not impinge directly in the machine direction MD. An air jet directed in the cross-direction CD, across the substrate, was also effective and eliminates air recirculation and stagnation regions in the flow regime adjacent the nip point, potentially allowing heating closer to the nip point. Heating the substrate as close to the nip point as practical reduces the cooling between heating and bonding, such that the maximum temperature experienced by the substrate is as low as practical for a given in-process temperature at the sonotrode. This enables having the in-process temperature as close to the melting temperature range Tm (or Tg, dependent upon polymer) as practical, without adverse quality effects from localized burn-through and thermal distortion.

When the portion 210 of the substrate 200 is pre-heated, it may be beneficial for the method to further comprise hindering or blocking some or all of the thermal energy from reaching the source of vibration energy 402, other portions 212 of the substrate 200, and/or the nip 800. To hinder or to block some or all of the thermal energy from reaching the source of vibration energy 402, one or more heat shields 900 may be employed. The heat shields 900 may include a top heat shield 902, a bottom heat shield 906, and/or an upwardly extending heat shield 910. The heat shields 900 may be configured to hinder or to block heat across an entire surface thereof or to allow heat, to pass through only one or more portions of a surface thereof. For example, the top heat shield 902 may have a surface defining a central aperture 904 to allow heat to pass therethrough. Similarly, the bottom heat shield 906 may have a surface defining a central aperture 908 to allow heat to pass therethrough. The method may also include cooling the first device 300 and/or the second device 400.

One or more moveable heat shield, which intermittently blocks heat to the substrate intermediate the patches, may be used. The hot air blast may be intermittent, partially blocked, redirected, turned away, or with a lower temperature to avoid heating the region intermediate the patches. The air temperature of the pre-heat may be chosen to be near but below the melting temperature of the substrate, to avoid burn-through and holes. A coating of additional material, such as applied by a slot coater, may be intermittently applied to the substrate to prevent burn-through between patches. Further, according to various embodiments it is possible to use a heat shield to prevent localized heating exceeding a desirable range. The heat shields may be uniform, or may mask off regions in the cross machine direction so only the desired patch is exposed. According to various embodiments, upper and lower heat thermal insulator shields may be disposed intermediate hot air nozzles and the substrate. Cutouts direct the hot air to the CD region where hooks will be formed, while protecting the rest of the substrate from heat affects. The heat shields extend adjacent the contact region between sonotrode and anvil roll. The sonotrode side heat shield may be combined with a sonotrode heat shield. A hold down plate, also known as a heat shield, may also serve to smooth the substrate. The heat tends to induce thermal distortions in the substrate. Contraction or expansion of fibers leads to shear stresses in the material, which leads to planar buckling. Machine operators may refer to these as wrinkles. The wrinkles tend to become trapped in the material at molding and may be a quality defect.

It has further been discovered that, particularly when the portion 210 of the substrate 200 is pre-heated, it may be beneficial for the method to comprise contacting the substrate 200 with a substrate spreader 700 upstream of the nip 800 to reduce or to mitigate fold over or wrinkles in the substrate 200. It may also be beneficial to convey the substrate 200 into contact with an idler 702 upstream of the substrate spreader 700. In addition to maintaining the temperature below the melting temperature of the portion 210 of the substrate 200 until the portion 210 of the substrate 200 is conveyed through the nip 800, it may be beneficial for various embodiments of the method to comprise maintaining the portion 210 of the substrate 200 free from contact with the first device 300 until the portion 210 of the substrate 200 is proximate to the nip 800. Limiting contact with the first device 300 may further aid in mitigating fold over or wrinkles in the substrate 200.

The heating of an anisotropic non-woven tends to result in thermal distortion and warpage, which may be observed as wrinkles and foldovers. These changes may interfere with the patch formation. Various embodiments may, therefore, employ a passive spreading device 700, in the form of a round bar, to flatten the substrate immediately upstream of the heating point. For example, a spreading device 700 adjacent the nip 800 may be employed. The spreading device 700 may be any suitable size and shape, for example a 12mm diameter cylindrical steel rod, with a 10-20° wrap angle.

The non-woven sheet may slide on the substrate spreader 700, such as a stationary bar. In this sliding configuration with low coefficient of friction, hoop stresses of the non-woven sheet wrapped on the bar may cause wrinkles in the substrate to flatten. One or more upstream roller(s) 702, 704 may also be employed upstream of the bar. The rollers 702, 704 may provide moment support, so localized tension variations across the CD and localized in-plane bending moments in the substrate may not propagate upstream, which may result in wrinkles, mistracking of the entire substrate and/or out of plane deformations. It is to be appreciated that a variety of suitable spreading devices may be utilized, including but not limited to flat bars, deadplates, bow bars, air bars, deformable brush rollers, stepped rollers, concave or V-shaped rollers, arco rollers, deformable element rollers, Mt. Hope rollers, tentering rollers, or a number of other devices known in the industry. Protrusions or recessed areas on the anvil roll may have a higher or lower surface velocity that the average velocity of the anvil roll. Such radius differences may be minimized to maintain a flat substrate. The sonotrode may be narrower than the non-woven substrate and may only be substantially similar in width to the patch region of the anvil roll. The sonotrode may cause tension, compression, and shear stresses in the non-woven substrate localized to a CD region. These areas may result in locally higher or lower machine direction tensile stresses, and the MD longitudinal stresses may be compressive in some regions. These locally varying stresses may lead to shear tresses and wrinkles in the substrate as discussed above. While protrusions may be required in a patch region to operatively engage the sonotrode, the protrusion heights may be minimized. Recessed areas may be minimized. The CD width of the sonotrode may be minimized, to enable the anvil roll surface radius and surface velocity adjacent the patch to match the patch region radius and surface velocity. The anvil roll CD regions adjacent the patch, particularly outboard the patch, may be increased radius to generate an in-plane moment, which may act to spread the substrate. Wrinkles, holes, tears and other defects may thus be mitigated.

Figure 1 is an example according to various embodiments illustrating a perspective view of an apparatus 100 for altering a portion of a substrate 200. The substrate 200 may be conveyed through the apparatus in a machine direction MD. As shown the substrate 200 is formed from a plurality of layers 216. The apparatus 100 comprises a first device 300, a second device 400, an upper heat source 500, a lower heat source 600, a substrate spreader 700, and a nip 800 formed between the first device 300 and the second device 400.

The first device 300 is an anvil roll 312, having an outer surface 302. The outer surface 302 comprises an indented portion 304 comprising a plurality of patterns 306. Each pattern 306 comprises a plurality of recesses or indentations 308. The second device 400 comprises a source of vibration energy 402, which is a rotating source of vibration energy 404, more specifically a rotary sonotrode 406. The first device 300 may rotate at a first velocity and in a first direction. The second device 400 may rotate at a second velocity and in a second direction. The first velocity and the second velocity may be variable and may be the same or different. The first direction and the second direction may be variable and may be the same or different.

The upper heat source 500 comprises an upper exhaust nozzle 502 aligned with and directing air toward a top surface 206 of the substrate 200. The lower heat source 600 comprises an lower exhaust nozzle 602 aligned with and directing air toward a bottom surface 208 of the substrate 200. The upper heat source 500 and the lower heat source 600 may selectively heat a portion 201 of the substrate 200 located between the upper exhaust nozzle 502 and the lower exhaust nozzle. Other portions 212 of the substrate may be heated to a lesser degree or may not be heated at all by the air from the upper heat source 500 and the lower heat source 600.

The apparatus 100 also includes a substrate spreader 700, a first idler 704, a second idler 706, and an outlet roller 706. The substrate 200 may be conveyed between the first idler and the second idler 706, which may be spaced to provide tension to the substrate 200 and to increase the distance that the substrate 200 must travel before reaching the substrate spreader 700. The substrate spreader 700 is a cylindrical bar extending in a cross-direction CD, which may add tension to the substrate immediately prior to the application of heat from the upper heat source 500 and the lower heat source 600, which may use substrate hoop stress to pull the substrate into a flat disposition adjacent the spreader bar, to mitigate fold-over and wrinkling of the substrate 200 upon being heated. The outlet roller 706 may maintain tension on the substrate as it exits the nip 800. The resulting substrate 202 may comprise a plurality of patches 204 suitable for use in a touch fastener. The patches may comprise a plurality of projections or hooks 214. The patches may be formed in the nip when the heated substrate is pressed into the recesses 308 of the patterns 306 on the first device 300 by the second device 400.

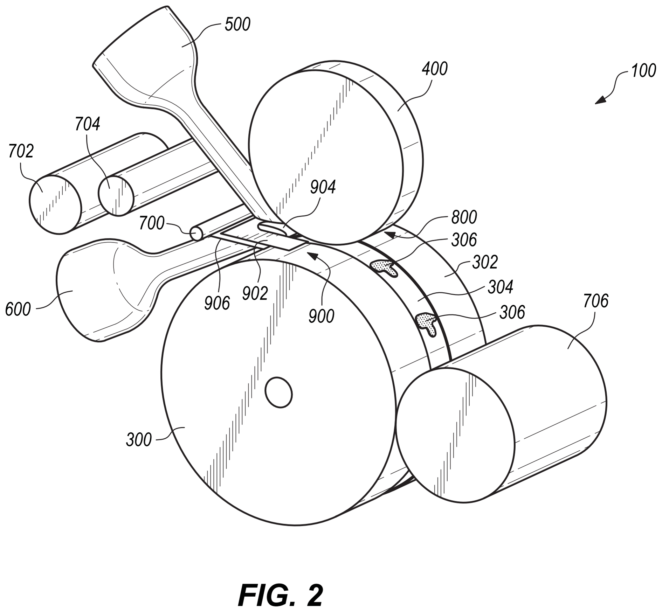

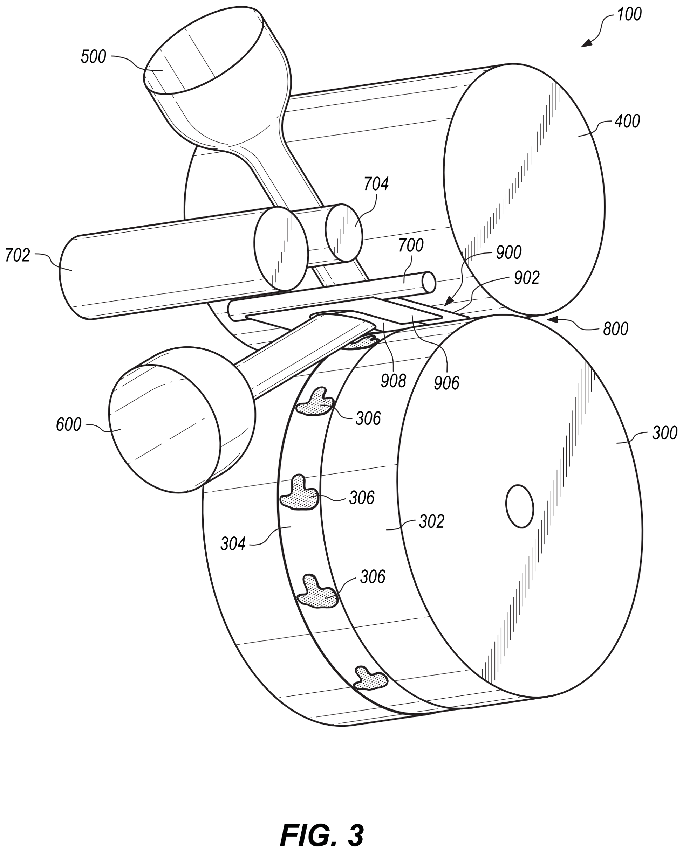

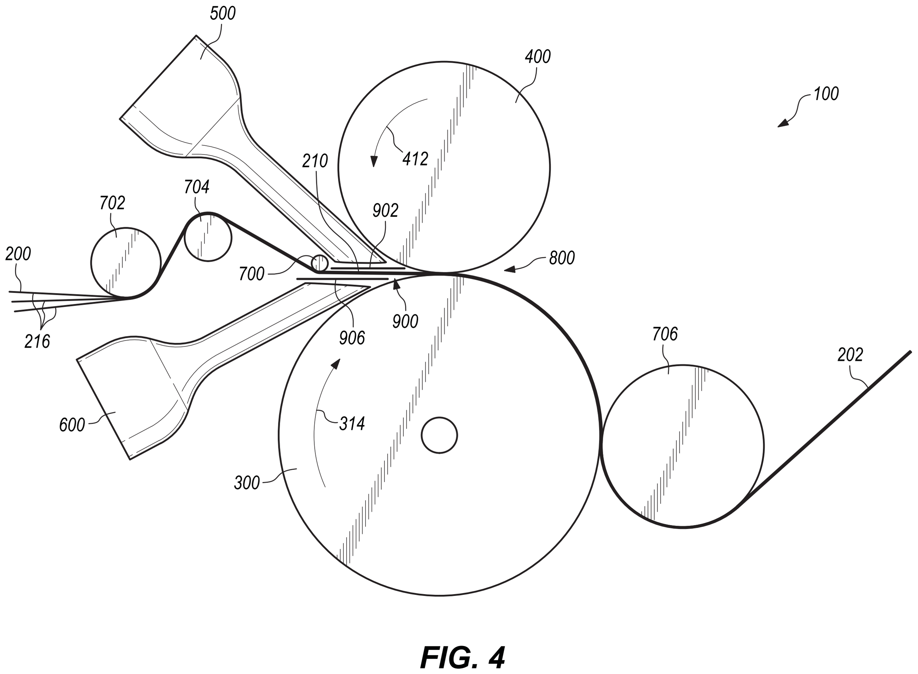

Figures 2 - 4 are examples according to various embodiments illustrating views of an apparatus 100 for altering a portion of a substrate 200 comprising the same or similar features as shown in Figure 1, but with the addition of heat shields 900. The heat shields 900 comprise a top heat shield 902 and a bottom heat shield 906. Figure 2 provides a perspective view of the apparatus 100, showing the top heat shield 902, having a central aperture 904. Figure 3 provides a bottom perspective view of the apparatus 100, showing the bottom heat shield 906, have a central aperture 908. Figure 4 provides a cross-sectional view of the apparatus 100.

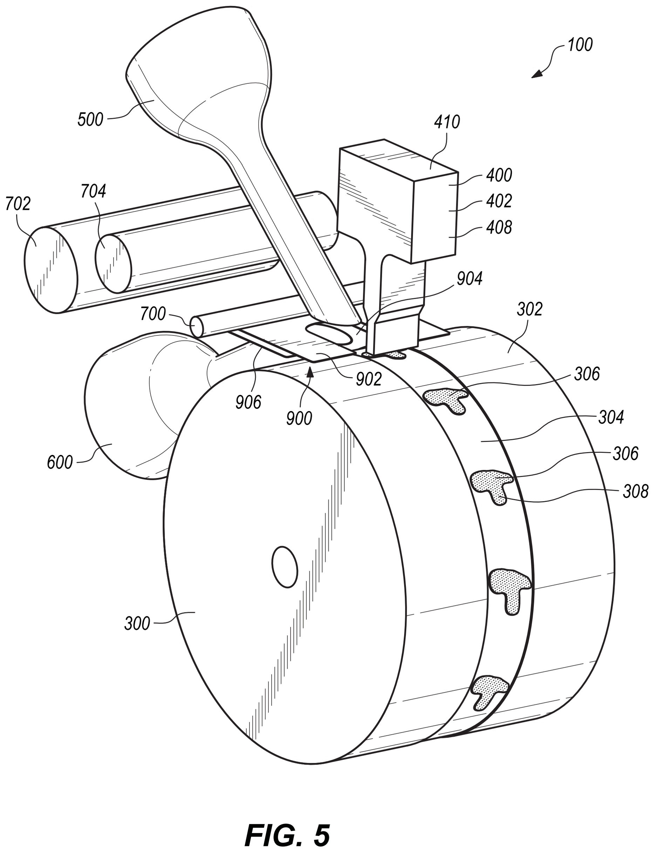

Figure 5 is an example according to various embodiments illustrating a perspective view of an apparatus 100 for altering a portion of a substrate 200 comprising the same or similar features as shown in Figure 1, but with the addition of heat shields 900 and the source of vibration energy 402 is a stationary source of vibration energy 410 instead of a rotating source of vibration energy 404. More specifically, the stationary source of vibration energy 410 is a blade sonotrode 408. The heat shields 900 comprise a top heat shield 902 and a bottom heat shield 906.

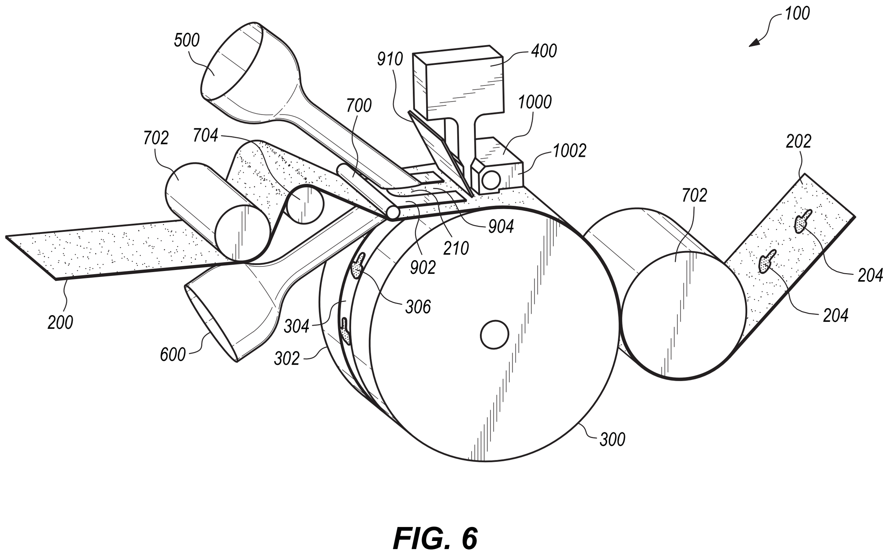

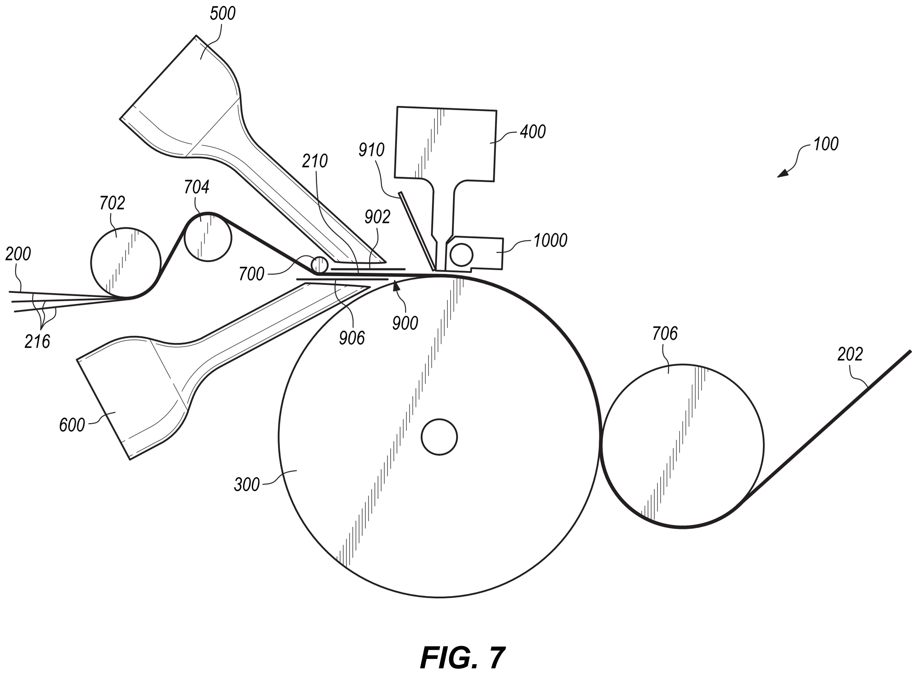

Figure 6 and 7 are an example according to various embodiments illustrating views of an apparatus 100 for altering a portion of a substrate 200 comprising the same or similar features as shown in Figure 5, but the heat shields 900 comprise a top heat shield 902, a bottom heat shield 906, and an upwardly extending heat shield 910 and the apparatus comprise a cooling apparatus 1000. More specifically, the cooling apparatus 1000 is a cooling block 1002. Figure 7 provides a cross-sectional view of an apparatus 100.

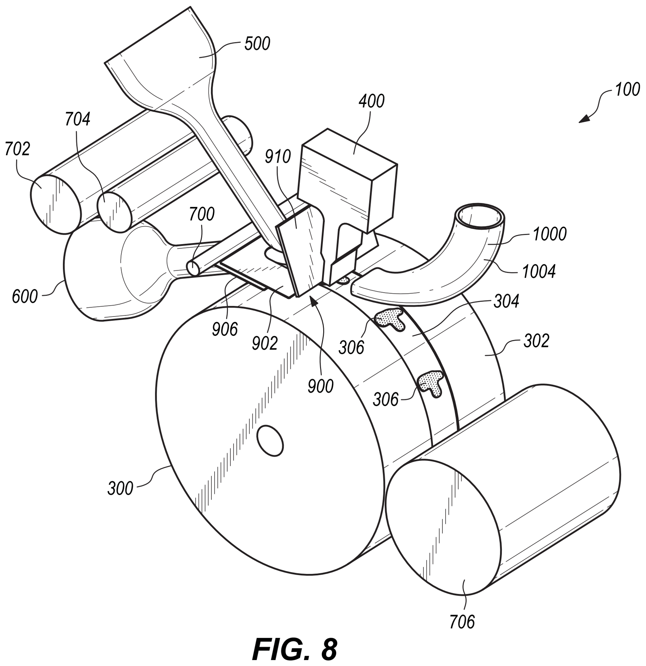

Figure 8 is an example according to various embodiments illustrating a perspective view of an apparatus 100 for altering a portion of a substrate 200 comprising the same or similar features as shown in Figures 7 and 8, but the cooling apparatus 1000 comprises a cooling blower 1004 instead of a cooling block 1002.

The rotary sonotrode tends to heat up during use. As the sonotrode heats up, the diameter changes, which may change the compression force between the sonotrode and the anvil roll. The gap between the first device and the second device at the nip also changes. In some cases, the feedback control loop is not able to compensate for the thermal growth, or the system must be run at a high contact force where the sensors cannot read. According to various embodiments, a cooling air jet may be sufficient to prevent overheating. Preferably, this cooling jet is below ambient, such as from the cold air port of a VORTEC^{®} nozzle connected to 10-60 psi compressed air. The cooling air blast must not interfere with the hot air blast on the substrate. As such, the cooling air blast may directed along a side opposed to the nip point (e.g. cold air to top, nip on bottom) and downstream of the sonotrode (e.g. facing downstream, clockwise airstream for counter-clockwise rotating rotary sonotrode), away from the heating air blast(s). Without being bound by theory, it is hypothesized that due to the Coanda effect, the cold air blast may envelope the anvil for about 20-90°. In another embodiment with a vented, blade sonotrode, cooling air may be directed upstream in the machine direction through a plurality of slots or cavities in the blade sonotrode. This cooling air may be close to 0°C or colder. The pre-heating air into the sonotrode may be 135-165°C. A sonotrode heat shield may be interposed between the air stream, to avoid unintended cooling of the preheated NW and/or heating of the sonotrode.

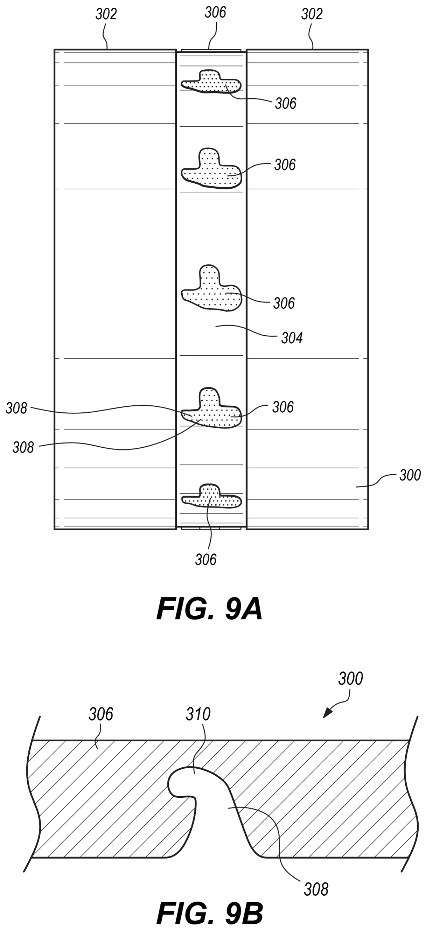

Figure 9A is an example according to various embodiments illustrating a side view of a first device 300 comprising a plurality of patterns 306 having recesses or indentations 308. The recesses or indentations 308 are formed in an indented portion 304 of the outer surface 302 of the first device 300, which may be an anvil roll 312.

Figure 9B is an example according to various embodiments illustrating a cross-sectional view of a recess 308 in one of the patterns 306, showing a shape 310 suitable for forming a projection or a hook 214 for use in a touch fastener.

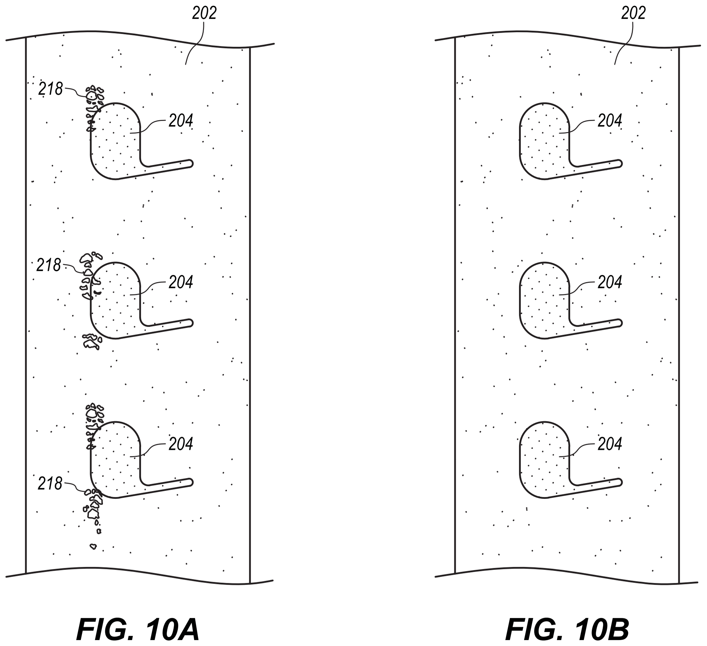

Figures 10A and 10B show examples of resulting sheets 202. Figure 10A is a comparative example illustrating a resulting sheet 202 having a plurality of patches 204, but also a plurality of burnt-through portions 218. Figure 10B is an example according to various embodiments illustrating a resulting sheet 202 having a plurality of patches 204, suitable for use as touch fasteners.

It is to be appreciated that the methods according to various embodiments may be executed with other rotary ultrasonic units, potentially at lower forces. Line speed might be reduced. Pattern width might be reduced. Multiple sonotrodes could be used in series. The sonotrodes in series may be of dissimilar types. For example, a rotary sonotrode could be used as a pre-processor, to create a film path and nubs without holes, with subsequent blade sonotrode as a finishing operation. These could be on separate or preferably a common anvil roll.

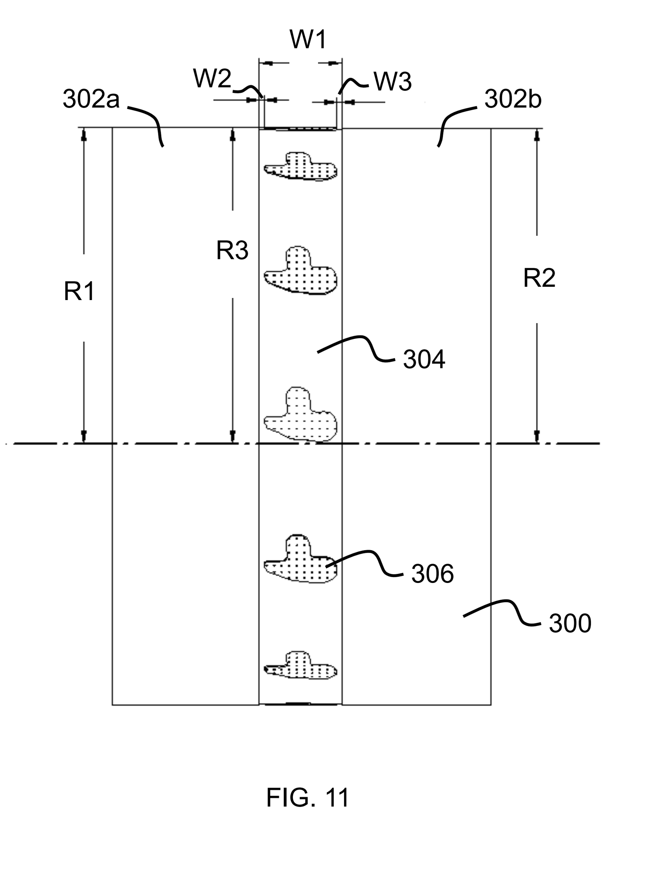

Figure 11 is an example according to various embodiments illustrating a side view of a first device 300 as shown in Figure 9A, showing several dimensions. A first portion 302a of the outer surface 302 of the first device 300 may have a first radius R1. A second portion 302b of the outer surface 302 of the first device may have a second radius R2. The first radius R1 and the second radius R2 may be the same or different. According to various embodiments, the second radius R2 may be less than the first radius R1 by about 0.1% to about 0.5%, or about 0.2% to about 0.4% or about 0.1 to about 0.3% or about 0.3% to about 0.5%. The pattern 306 may be disposed at a third radius R3. The third radius R3 may be the same as or different than either the first radius R1 or the second radius R2. For example, according to various embodiments, the third radius R3 may be less than the first radius R1 by about 0.05% to about 0.1%, or about 0.06% to about 0.9%, or about 0.7% to about 0.8%.

Still referring to Figure 11, the indented portion 304 of the outer surface 302 of the first device 300 may have a first width W1. The first width W1 may be less than the width of the pattern 306, such that the pattern is spaced away from the first portion 302a by a second width W2 and away from the second portion 302b by a third width W3.

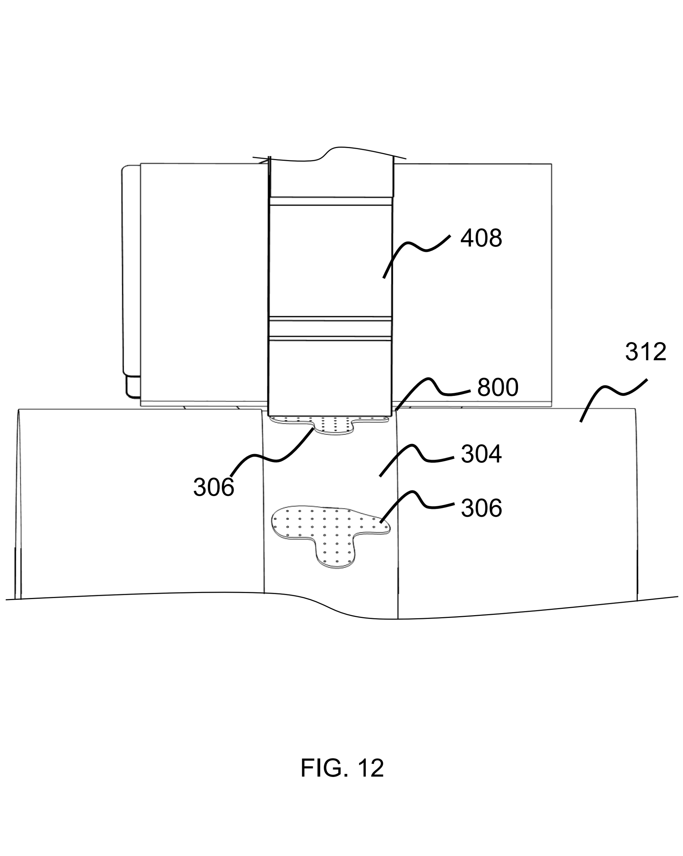

In embodiments utilizing rotary sonotrodes, it has been discovered that higher amplitudes generally result in better hook formation, without the limitations of burn through or hole formation observed with blade sonotrode. To allow for higher amplitudes, the sonotrode may be just slightly wider than the pattern. Figure 12 is an example according to various embodiments showing a blade sonotrode 408 that is only slightly wider than the pattern 306, such that the blade sonotrode 408 may fit into the indented portion 304 of the anvil roll 312. A narrow rotary sonotrode (not shown) may be provided with a similar width and may be positioned in a similar manner at the nip 800 such that the rotary sonotrode is disposed within the indented portion 304 of the anvil roll 312. According to various embodiments, the second device, whether a blade sonotrode or a rotary sonotrode may have a width that is just slightly wider than the width of the pattern 306. For example, the second device 300 may be 45mm wide for a 40mm pattern 306 rather than 85 mm wide for a 40mm pattern 306. The narrower working surface may also enable a higher amplitude.

It was discovered that a blade sonotrode may elongate the patch region in the machine. While the recesses 308 of the first device 300 have positions that may be fixed, the transition regions adjacent leading and trailing edges of the patch may distort. For example, a nominally 30mm patch may be 30.5 or 31.0mm long when made by a blade sonotrode. This increased machine direction MD length may create a slackness in the substrate at stripping, which may cause a tenting effect. Tenting in this usage means an machine direction MD length is increased in a cross-direction CD region of the patch and/or a cross-direction CD length of the patch is increased in a machine direction MD region of the patch. The tenting may often combine both machine direction MD and cross-direction CD distortions. Testing with high-speed video indicated a tenting effect at stripping, which is more noticeable with longitudinally and/or laterally slack substrates. Fractured hooks at stripping are highly correlated with the longitudinal and/or lateral slackness. The hooks are pulled at a larger incidence angle relative the anvil roll surface when the substrate is slack, which may cause hooks to fracture. This extraction angle may superimpose a stress field due to rotation on the existing stress field due to kinematic constraints and substrate tension at extraction. Forward facing hooks, where a cantilevered hook end extends in a downstream direction, may be even more susceptible. The larger incidence angle at extraction may also cause a snapping where several cross-direction CD rows of hooks release near concurrently from the anvil roll, which may be due to a larger component of the substrate tension normal the anvil roll due to the increased substrate angle. During testing, this snapping effect was commonly seen at the trailing edge of the patch.

### Examples/Combinations:

1. A method of altering a portion **(210)** of a substrate **(200)** comprising:
   providing a first device **(300)** comprising an outer surface **(302);**
   providing a second device **(400)** comprising a rotating source of vibration energy **(404);**
   forming a nip **(800)** between the rotating source of vibration energy **(404)** and the outer surface **(302);**
   conveying the substrate **(200)** through the nip **(800);**
   altering the portion **(210)** of the substrate **(200)** in the nip **(800)** using the rotating source of vibration energy **(404),** wherein the rotating source of vibration energy **(404)** applies a lineal force of about 1 kN to about 5 kN per 25 mm of operative contact width, wherein the rotating source of vibration energy **(404)** has an about 20 to about 60 micron zero-to-peak sinusoidal amplitude, and wherein the rotating source of vibration energy **(404)** applies about 15 kHz to about 29 kHz of vibration energy to the substrate.
2. The method of Paragraph 1, wherein the rotating source of vibration energy **(404)** applies vibration energy to the substrate **(200)** intermittently.
3. The method of Paragraph 1 or Paragraph 2, comprising imparting thermal energy to the portion **(210)** of the substrate **(200)** upstream of the nip **(800)** to heat the portion **(210)** of the substrate **(200)** to a temperature below a melting temperature of the portion **(210)** of the substrate **(200),** wherein the temperature below the melting temperature of the portion **(210)** of the substrate **(200)** is in a range of about 90 degrees C to about 160 degrees C.
4. The method of Paragraph 0, comprising hindering the thermal energy from reaching the rotating source of vibration energy **(404),** other portions **(212)** of the substrate **(200),** and/or the nip **(800).**
5. The method of any one of Paragraphs 1 to 4, comprising rotating the first device **(300)** at a first surface velocity different than a second surface velocity of the rotating source of vibration energy **(404).**
6. The method of Paragraph 5, wherein the first surface velocity is variable, or wherein the second surface velocity is variable.
7. The method of Paragraph 5, wherein the first surface velocity is higher than the second surface velocity.
8. The method of Paragraph 5, wherein the first surface velocity is lower than the second surface velocity.
9. The method of any one of Paragraphs 1 to 8, comprising cooling the rotating source of vibrational energy **(404)** or the first device **(300).**
10. The method of any one of Paragraphs 1 to 9, wherein the second device **(400)** is a rotary sonotrode **(406),** and wherein the vibration energy is ultrasonic energy.
11. The method of any one of Paragraphs 1 to 10, comprising providing a plurality of recesses **(308)** in the outer surface **(302)** of the first device **(300),** wherein the recesses **(308)** have a shape **(310)** configured to produce projections **(214)** suitable for use in a touch fastener.
12. The method of any one of Paragraphs 1 to 11, wherein the substrate **(200)** comprises more than one layer **(216)** or more than one material.
13. A method of altering a portion **(210)** of the substrate **(200)** comprising:
   providing a first device **(300)** comprising an outer surface **(302);**
   providing a second device **(400)** comprising a source of vibration energy **(402);**
   forming a nip **(800)** between the source of vibration energy **(402)** and the outer surface **(302);**
   applying thermal energy to a portion **(210)** of the substrate upstream of the nip **(800)** to raise a temperature of the portion **(210)** of the substrate **(200)** to a temperature below a melting temperature of the portion **(210)** of the substrate **(200),** wherein the temperature below the melting temperature of the portion **(210)** of the substrate **(200)** is in the range of about 90 degrees C to about 160 degrees C;
   conveying the substrate **(200)** through the nip **(800);** and
   applying vibration energy from the source of vibration energy **(402)** to the substrate **(200)** in the nip **(800)** to alter the portion **(210)** of the substrate **(200).**
14. The method of Paragraph 13, wherein the source of vibration energy **(402)** is a rotary sonotrode **(406).**
15. The method of Paragraph 13 or Paragraph 14, wherein the rotary sonotrode **(406)** applies a lineal force of about 1 kN to about 5 kN per 25 mm of operative contact width, wherein the rotary sonotrode **(406)** has a zero-to-peak sinusoidal amplitude of about 20 to about 60 microns, and wherein the rotary sonotrode **(406)** applies about 15 kHz to about 29 kHz of vibration energy to the substrate **(200).**
16. The method of any one of Paragraphs 13 to 15, wherein the source of vibration energy **(402)** is a blade sonotrode **(408).**
17. The method of any one of Paragraphs 13 to 16, comprising rotating the first device **(300)** at a first surface velocity.
18. The method of Paragraph 17, comprising rotating the source of vibration energy **(402)** at a second surface velocity that is different than the first surface velocity, and wherein the first surface velocity or the second surface velocity is variable.
19. The method of any one of Paragraphs 13 to 18, comprising applying the thermal energy to a first side **(206)** of the substrate **(200).**
20. The method of Paragraph 19, comprising applying the thermal energy to a second side **(208)** of the substrate **(200).**
21. The method of any one of Paragraphs 13 to 20, comprising contacting the substrate **(200)** with a substrate spreader **(700)** upstream of the nip **(800)** to reduce fold over or wrinkles in the substrate **(200).**
22. The method of any one of Paragraphs 13 to 21, comprising providing a plurality of recesses **(308)** in the outer surface **(302)** of the first device **(300),** wherein the recesses **(308)** have a shape **(310)** configured to produce projections **(214)** suitable for use in a touch fastener.
23. The method of any one of Paragraphs 13 to 22, comprising hindering the thermal energy from reaching the source of vibration energy **(402),** other portions **(212)** of the substrate **(200),** and/or the nip **(800).**
24. The method of any one of Paragraphs 13 to 23, wherein the vibration energy is applied to the substrate **(200)** intermittently.
25. The method of any one of Paragraphs 13 to 24, comprising:
   maintaining the temperature below the melting temperature of the portion **(210)** of the substrate **(200)** until the portion **(210)** of the substrate **(200)** is conveyed through the nip **(800);** and
   maintaining the portion **(210)** of the substrate **(200)** free from contact with the first device **(300)** until the portion **(210)** of the substrate **(200)** is proximate to the nip **(800).**
26. A method of altering a portion **(210)** of a substrate **(200)** comprising:
   providing a first device **(300)** comprising an outer surface **(302);**
   providing a second device **(400)** comprising a source of vibration energy **(402);**
   forming a nip **(800)** between the source of vibration energy **(402)** and the outer surface **(302);**
   conveying the substrate **(200)** into contact with a substrate spreader **(700)** upstream of the nip **(800)** to mitigate fold over or wrinkles in the substrate **(200);**
   conveying the substrate **(200)** through the nip **(800);** and
   applying vibration energy from the source of vibration energy **(402)** to the substrate **(200)** in the nip **(800)** to alter the portion **(210)** of the substrate **(200).**
27. The method of Paragraph 26, comprising:
   applying thermal energy to a portion **(210)** of the substrate **(200)** upstream of the nip **(800)** to raise a temperature of the portion **(210)** of the substrate **(200)** to a temperature below a melting temperature of the portion **(210)** of the substrate **(200);**
   wherein the thermal energy has a temperature in the range of about 90 degrees C to about 190 degrees C;
   maintaining the temperature of the portion **(210)** of the substrate **(200)** until the portion **(210)** of the substrate **(200)** is conveyed through the nip **(800).**
28. The method of Paragraph 27, comprising hindering the thermal energy from reaching the source of vibration energy **(402),** other portions **(212)** of the substrate **(200),** and/or the nip **(800).**
29. The method of any one of Paragraphs 26 to 28, wherein the source of vibration energy **(402)** is a rotary sonotrode **(406),** and wherein the first device **(300)** is an anvil roll **(308),** comprising:
   rotating the anvil roll **(308)** at a first surface velocity; and
   rotating the rotary sonotrode **(406)** at a second surface velocity;
   wherein the first surface velocity is different than the second surface velocity.
30. The method of Paragraph 29, wherein the first surface velocity or the second surface velocity is variable.
31. The method of any one of Paragraphs 26 to 30, wherein the source of vibration energy **(402)** is a blade sonotrode **(408).**
32. The method of any one of Paragraphs 26 to 31, comprising conveying the substrate **(200)** into contact with an idler **(702)** upstream of the substrate spreader **(700).**
33. The method of any one of Paragraphs 26 to 32, wherein the vibration energy is applied to the substrate **(200)** intermittently.
34. The method of any one of Paragraphs 26 to 33, wherein the source of vibration energy **(402)** is a rotary sonotrode **(406),** wherein the rotary sonotrode **(406)** applies a lineal force of about 1 kN to about 5 kN per 25 mm of operative contact width, wherein the rotary sonotrode **(406)** has an about 20 to about 60 micron zero-to-peak sinusoidal amplitude, and wherein the rotary sonotrode **(406)** applies about 15 kHz to about 29 kHz of vibration energy to the substrate **(200).**

### Examples/Combinations:

1. A method of altering a portion **(210)** of the substrate **(200)** comprising:
   providing a first device **(300)** comprising an outer surface **(302);**
   providing a second device **(400)** comprising a source of vibration energy **(402);**
   forming a nip **(800)** between the source of vibration energy **(402)** and the outer surface **(302);**
   applying thermal energy to a portion **(210)** of the substrate upstream of the nip **(800)** to raise a temperature of the portion **(210)** of the substrate **(200)** to a temperature below a melting temperature of the portion **(210)** of the substrate **(200),** wherein the temperature below the melting temperature of the portion **(210)** of the substrate **(200)** is in the range of about 90 degrees C to about 160 degrees C;
   conveying the substrate **(200)** through the nip (800); and
   applying vibration energy from the source of vibration energy **(402)** to the substrate **(200)** in the nip **(800)** to alter the portion **(210)** of the substrate **(200).**
2. The method of Paragraph 1, wherein the source of vibration energy **(402)** is a rotary sonotrode **(406).**
3. The method of Paragraph 2, wherein the rotary sonotrode **(406)** applies a lineal force of about 1 kN to about 5 kN per 20 mm of operative contact width, wherein the rotary sonotrode **(406)** has a peak-to-peak sinusoidal amplitude of about 20 to about 60 microns, and wherein the rotary sonotrode **(406)** applies about 15 kHz to about 29 kHz of vibration energy to the substrate **(200).**
4. The method of Paragraph 1, wherein the source of vibration energy **(402)** is a blade sonotrode **(408).**
5. The method of any one of Paragraphs 1 to 4, comprising applying the thermal energy to a first side **(206)** of the substrate **(200).**
6. The method of Paragraph 5, comprising applying the thermal energy to a second side **(208)** of the substrate **(200).**
7. The method of any one of Paragraphs 1 to 6, comprising contacting the substrate **(200)** with a substrate spreader **(700)** upstream of the nip **(800)** to reduce fold over or wrinkles in the substrate **(200).**
8. The method of any one of Paragraphs 1 to 7, comprising providing a plurality of recesses **(308)** in the outer surface **(302)** of the first device **(300),** wherein the recesses **(308)** have a shape **(310)** configured to produce projections **(214)** suitable for use in a touch fastener.
9. The method of any one of Paragraphs 1 to 8, comprising hindering the thermal energy from reaching the source of vibration energy **(402),** other portions **(212)** of the substrate **(200),** and/or the nip **(800).**
10. The method of any one of Paragraphs 1 to 9, wherein the vibration energy is applied to the substrate **(200)** intermittently.
11. The method of any one of Paragraphs 1 to 10, comprising:
   maintaining the temperature below the melting temperature of the portion **(210)** of the substrate **(200)** until the portion **(210)** of the substrate **(200)** is conveyed through the nip **(800);** and
   maintaining the portion **(210)** of the substrate **(200)** free from contact with the first device **(300)** until the portion **(210)** of the substrate **(200)** is proximate to the nip **(800).**
12. The method of any one of Paragraphs 1 to 11, wherein the substrate **(200)** comprises more than one layer **(216)** or more than one material.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present disclosure have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A method of altering a portion **(210)** of a substrate **(200)** comprising:
providing a first device **(300)** comprising an outer surface **(302);**
providing a second device **(400)** comprising a rotating source of vibration energy **(404);**
forming a nip **(800)** between the rotating source of vibration energy **(404)** and the outer surface **(302);**
conveying the substrate **(200)** through the nip **(800);**
altering the portion **(210)** of the substrate **(200)** in the nip **(800)** using the rotating source of vibration energy **(404),** wherein the rotating source of vibration energy **(404)** applies a lineal force of about 1 kN to about 5 kN per 25 mm of operative contact width, wherein the rotating source of vibration energy **(404)** has an about 20 to about 60 micron zero-to-peak sinusoidal amplitude, and wherein the rotating source of vibration energy **(404)** applies about 15 kHz to about 29 kHz of vibration energy to the substrate.

2. The method of Claim 1, wherein the rotating source of vibration energy **(404)** applies vibration energy to the substrate **(200)** intermittently.

3. The method of Claim 1 or Claim 2, comprising imparting thermal energy to the portion **(210)** of the substrate **(200)** upstream of the nip **(800)** to heat the portion **(210)** of the substrate **(200)** to a temperature below a melting temperature of the portion **(210)** of the substrate **(200),** wherein the temperature below the melting temperature of the portion **(210)** of the substrate **(200)** is in a range of about 90 degrees C to about 160 degrees C.

4. The method of Claim 3, comprising hindering the thermal energy from reaching the rotating source of vibration energy **(404),** other portions **(212)** of the substrate **(200),** and/or the nip **(800).**

5. The method of any one of Claims 1 to 4, comprising rotating the first device **(300)** at a first surface velocity different than a second surface velocity of the rotating source of vibration energy **(404).**

6. The method of Claim 5, wherein the first surface velocity is variable, or wherein the second surface velocity is variable.

7. The method of Claim 5, wherein the first surface velocity is higher than the second surface velocity.

8. The method of Claim 5, wherein the first surface velocity is lower than the second surface velocity.

9. The method of any one of Claims 1 to 8, comprising cooling the rotating source of vibrational energy **(404)** or the first device **(300).**

10. The method of any one of Claims 1 to 9, wherein the second device **(400)** is a rotary sonotrode **(406),** and wherein the vibration energy is ultrasonic energy.

11. The method of any one of Claims 1 to 10, comprising providing a plurality of recesses **(308)** in the outer surface **(302)** of the first device **(300),** wherein the recesses **(308)** have a shape **(310)** configured to produce projections **(214)** suitable for use in a touch fastener.

12. The method of any one of Claims 1 to 11, wherein the substrate **(200)** comprises more than one layer **(216)** or more than one material.

13. The method of any one of Claims 1 to 12, comprising contacting the substrate **(200)** with a substrate spreader **(700)** upstream of the nip **(800)** to reduce fold over or wrinkles in the substrate **(200).**

14. The method of Claim 3, comprising applying the thermal energy to a first side **(206)** of the substrate **(200).**

15. The method of Claim 14, comprising applying the thermal energy to a second side **(208)** of the substrate **(200).**
